# EUROPÄISCHE PATENTSCHRIFT

(11) **EP 2 439 188 B1**
(45) Veröffentlichungstag und Bekanntmachung des Hinweises auf die Patenterteilung: **31.10.2012**
(21) Anmeldenummer: 10176810.9
(22) Anmeldetag: 15.09.2010
(51) Int. Cl.: C07C 69/40, C07C 69/44, C07C 67/08, C07C 59/13, C07C 217/12, C07C 229/08, C07C 309/23, A01N 31/04, A01N 37/02, A01N 41/04

(54) **p-Menthan-3,8-diolderivate und sie enthaltende Insektenschutzmittel**
p-Menthane-3,8-diol derivatives and insect repellents comprising them
Dérivés de menthane-3,8-diol et répulsifs contre les insectes comprenant ces dérivés

(43) Veröffentlichungstag der Anmeldung: 11.04.2012
(73) Patentinhaber: Selkis SAS, 92100 Boulogne-Billancourt (FR)
(72) Erfinder: Rataj-Nardello, Véronique, Prof., 59710 Pont-à-Marcq (FR); Touraud, Didier, Dr., 93051 Regensburg (DE); Kunz, Werner, Prof., 93051 Regensburg (DE); Drapereau, Jeremy, 92100 Boulogne-Billancourt (FR); Rose, Andreas, Dr., 93051 Regensburg (DE)
(74) Vertreter: HOFFMANN EITLE

(56) Entgegenhaltungen:
- WO-A1-2004/006674
- JP-A- 3 133 906

## Beschreibung

### Gebiet der Erfindung

Gegenstand der Erfindung sind neuartige *para*-Menthane-3,8-diolderivate, welche u.a. als Abwehrmittel gegen Arthropoden wie zum Beispiel Insekten eingesetzt werden können. Die neuen *para*-Menthane-3,8-diolderivate sind wasserlöslich und haben eine insektenabweisende Wirkung, die ähnlich der von bekannten Insektenschutzmitteln wie *para*-Menthan-3,8-diol (PMD) oder *N*,*N*-Diethyl-*m*-methylbenzamid (DEET) ist. Ferner haben die neuen *para*-Menthane-3,8-diolderivate die Eigenschaft, die Wasserlöslichkeit gängiger Insektenschutzmittel zu erhöhen.

### Stand der Technik

Wirkstoffe wie DEET (*N*,*N*-Diethyl-*m*-methylbenzamid), IR 3535 (Ethyl-3-[(acetyl)(butyl)amino]propanoat), KBR 3023 ((RS)-*sec*-butyl-(RS)-2-(2hydroxyethyl) piperidine-1-carboxylat), lcaridin (1-(1-Methylpropoxycarbonyl)-2-(2-hydroxyethyl)piperidin) oder PMD (*para*-Mentan-3,8-diol) werden oft in Produktformulierungen zum Insektenschutz benutzt, um für einen effektiven und lang anhaltenden Schutz vor blutsaugenden Arthropoden zu sorgen. Ein wesentlicher Nachteil dieser Wirkstoffe ist ihre geringe Wasserlöslichkeit. Aus diesem Grunde müssen der Produktformulierung organische Lösungsmittel, wie zum Beispiel Ethanol oder Propanol, und/oder Tenside zugesetzt werden, um die Wirkstoffe in einer wässrigen Phase zu lösen.

Es ist jedoch bekannt, dass das häufig als Co-Lösungsmittel benutzte Ethanol die Penetration der Wirkstoffe in die Haut signifikant erhöhen und beschleunigen kann. Dies hat einen Verlust an aktiver Substanz zur Folge und kann ebenso wie die eingesetzten Lösungsmittel zu Hautreizungen und allergischen Reaktionen führen. Aus diesem Grund werden in modernen Formulierungen Alkohole zum Teil durch Tenside ersetzt. Aber auch der Einsatz von Tensiden beherbergt Nachteile in Form von möglichen Hautirritationen oder allergischen Reaktionen, weshalb die Möglichkeit zum Verzicht auf ihren Einsatz wünschenswert ist.

Die Herstellung von Derivaten und Formulierungen von PMD hatte in der Vergangenheit meist das Ziel einer verlängerten Wirkdauer und beruhte auf der Annahme, dass die Wirkdauer üblicher lnsektenschutzmittel vor allem deshalb mit der Zeit nachlässt, weil sie relativ schnell verdampfen. Aufgrund dieser Überlegung wurde versucht, eine Verlängerung der Wirkdauer durch beschränkte Freisetzung der aktiven Substanz zu erreichen. So beschreiben EP 348 550 B und EP 502 119 B Formulierungen, in weichen die aktive Substanz in Mikrokapsein oder Liposphären eingebettet ist, US 5,015,570 A die Einbettung der aktiven Substanz in eine Kohlenhydratmatrix und EP 0274574 A und US 6180127 B die Beimischung eines Polymers. Auch Derivatisierungen zum Zwecke der Erhöhung der Wirkdauer wurden in Betracht gezogen. So beschreibt z.B. WO 2004/006674 die Herstellung von C₈₋₃₀ Estern von PMD. Ferner beschreibt JP 3 133906 A ein Schädlingsabwehrmittel, welches N,N-Diethyl-toluamid (DEET) und ein p-Menthanderivat , wie z.B. einen Monocarbonsäure- oder einen Sulfonsäureester von p-Mentan-3,8-cis-diol, enthält.

Diese Zusammensetzungen weisen jedoch immer noch den Nachteil auf, dass keine von ihnen wasserlöslich ist, bzw. eine hohe Wasserbeständigkeit sogar explizit erwünscht ist, und dass die Formulierungen daher Bestandteile enthalten, welche unter dem Gesichtspunkt der Hautverträglichkeit unerwünscht sind.

### Aufgabenstellung

In Anbetracht des Standes der Technik und der damit verbundenen Nachteile haben es sich die Erfinder zur Aufgabe gemacht, ein wasserlösliches Insektenschutzmittel zu entwickeln, bei dessen Formulierung auf den Einsatz von unerwünschten Zusatzstoffen, wie z.B. Alkohole oder Tenside, verzichtet werden kann, und welches eine Aktivität und Wirkdauer vergleichbar mit denen gängiger Insektenschutzmittel aufweist.

### Gegenstand der Erfindung

Die hier beschriebene Erfindung umfasst Verbindungen gemäß der folgenden Formel (1): 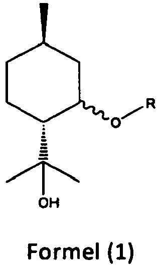 R ist eine gesättigte oder ungesättigte C₂-C₆ Alkyl- oder Acylgruppe, welche mit mindestens einer funktionellen Gruppe ausgewählt aus -CO₂M, -SO₃M, -PO₄M und -NH₃⁺X⁻ substituiert ist, wobei
M ein Wasserstoffatom oder ein Alkalimetall, insbesondere Na oder K, ist und
X⁻ ein Halogenid ist.

Ferner umfasst die Erfindung eine die erfindungsgemäße Verbindung umfassende wässrige Lösung.

Die Erfindung umfasst auch wässrige Lösungen, welche ein weiteres Insektenschutzmittel, wie z.B. *para*-Menthan-3,8-diol (PMD), *N*,*N*-Diethyl-*m*-methylbenzamid (DEET), 1-(1-Methylpropoxycarbonyl)-2-(2-hydroxyethyl)piperidin (Icaridin) und/oder Ethyl-3-[(acetyl)(butyl)amino]propanoat (IR3535), enthalten.

Die Erfindung umfasst ferner die im Vorgehenden beschriebenen wässrigen Lösungen, welche ferner ein oder mehrere Additive ausgewählt aus Konservierungsstoffen, Gelbildnern und Parfümen enthalten.

Die Erfindung umfasst weiterhin die Verwendung der erfindungsgemäßen Verbindungen oder wässrigen Lösungen als Insektenschutzmittel sowie die Verwendung der Verbindungen zur Erhöhung der Wasserlöslichkeit gängiger Insektenschutzmitteln, wie. z.B. PMD, DEET, Icaridin und/oder IR3535.

Zuletzt umfasst die Erfindung ein Verfahren zur Herstellung der erfindungsgemäßen Verbindungen durch Umsetzung einer Verbindung der Formel (2) (para-Menthan-3,8-diol) mit einer Verbindung R-A, wobei R eine gesättigte oder ungesättigte C₂-C₆ Alkyl- oder Acylgruppe darstellt, welche mit mindestens einer funktionellen Gruppe ausgewählt aus -CO₂M, -SO₃M, -PO₄M und -NH₃⁺X⁻ substituiert ist, M Wasserstoff oder ein Alkalimetall ist, X⁻ ein Halogenid ist und es sich bei A um ein Halogenatom, -OH oder -OTs (Ts = p-Toluolsulfonyl) handelt:

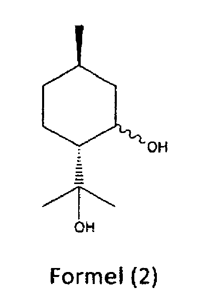

### Beschreibung der Figuren

Figur 1: Wirkdauer verschiedener Insektenschutzstoffe. (PMD und DEET in alkoholischer Lösung, PMD-S in wässriger Lösung).
Figur 2: Ternäres Phasendiagramm bei 25°C für das Dreikomponentensystem PMD/PMD-S/ Wasser (PMD-S = *para*-Menthane-3,8-diol Succinat). Die Zusammensetzungen sind in Gewichtsprozent angegeben. L ist der Bereich einphasiger Mischungen. N bezeichnet den Zusammensetzungsbereich mehrphasiger Mischungen.
Figur 3: Ergebnisse von Zytotoxizitätstests der hauptsächlichen Repellent-Wirkstoffe auf dem Markt.

### Beschreibung der Erfindung

Die Erfinder haben herausgefunden, dass PMD durch geeignete Veretherung oder Veresterung wasserlöslich gemacht werden kann. Somit wird durch die erfindungsgemäßen Verbindungen eine Formulierung auf Wasserbasis ohne Zugabe von unerwünschten Zusatzstoffen wie Alkohole und Tenside möglich.

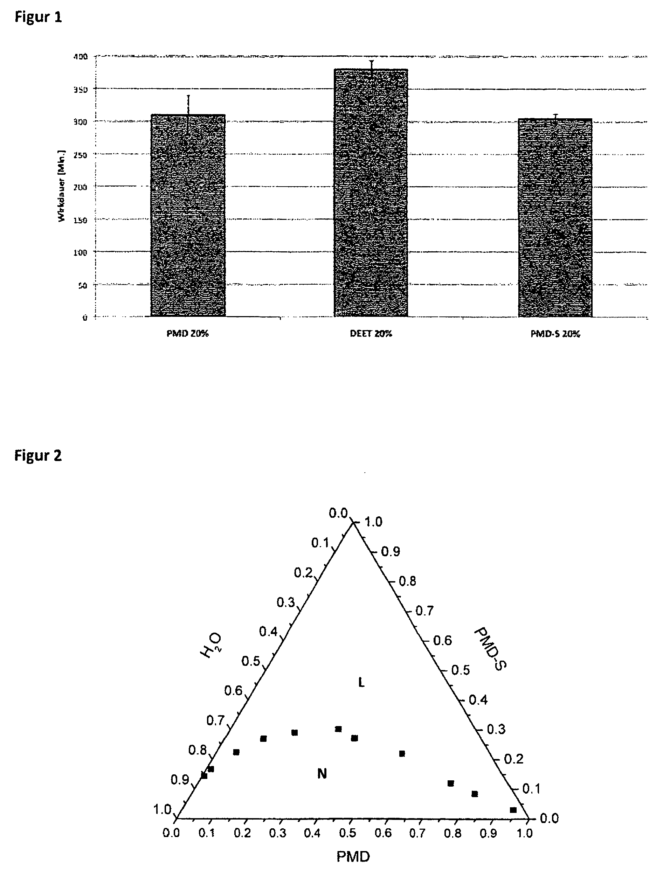

Ferner wurde bei der Prüfung der Insektenschutzwirkung erstaunlicherweise festgestellt, dass die erfindungsgemäßen Verbindungen eine nahezu unveränderte Wirkdauer im Vergleich zu PMD aufweisen (siehe Figur 1). Dieses unerwartete Ergebnis gewährleistet die Effektivität der erfindungsgemäßen Formulierungen.

Überraschenderweise wurde auch festgestellt, dass die erfindungsgemäßen Verbindungen die Eigenschaft aufweisen, die Wasserlöslichkeit gängiger Insektenschutzmittel, wie z.B. PMD, DEET, Icaridin und IR3535, zu erhöhen (siehe Figur 2). Somit, ergibt sich die Möglichkeit kostengünstigerer Formulierungen zum Insektenschutz, in weichen die erfindungsgemäßen Verbindungen in Kombination mit bekannten Wirkstoffen zum Einsatz kommen.

### Erfindungsgemäße Verbindungen

Bei den erfindungsgemäßen Verbindungen handelt es sich um Verbindungen mit der folgenden Struktur: 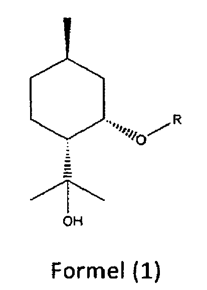 wobei R eine gesättigte oder ungesättigte C₂-C₆, Alkyl- oder Acylgruppe ist, welche mit mindestens einer funktionellen Gruppe ausgewählt aus ―CO₂M ―SO₃M, ―PO₄M und ―NH₃⁺X ⁻ substituiert ist,
M ein Wasserstoffatom oder ein Alkalimetall, insbesondere Na oder K, ist und
X⁻ ein Halogenid ist.

Spezielle Beispiele für die Gruppe R sind ―(CH₂)₂―SO₃Na, ―(CH₂)₂-NH₃⁺Cl⁻, ―CO―(CH₂)₂―SO₃Na, ―CO―(CH₂)₂― NH₃⁺Cl⁻, ―CO―(CH₂)₂―CO₂Na, ―(CH₂)₂―CO₂Na, ―CO―(CH₂)₃―CO₂Na und ―(CH₂)₃― CO₂Na

Bevorzugt sind Verbindungen, bei denen R eine mit ―CO₂ oder ―SO₃M substituierte C₂-C₆ Acylgruppe ist; insbesondere bevorzugt sind Gruppen der Formel ―CO―(CH₂)ₙ―CO₂M, worin n einen Wert von 1 bis 4 annehmen kann, wobei eine Gruppe der Formel ―CO―(CH₂)₂―CO₂Na am bevorzugtesten ist.

M ist bevorzugter Weise Na oder K, am bevorzugtesten Na.

### Physikalische Eigenschaften

Die erfindungsgemäßen Verbindungen weisen eine hohe Wasserlöslichkeit (über 50 g/L bei 25ºC) auf. In Verbindung mit ihrer Schutzaktivität gegen Insekten macht diese Eigenschaft die erfindungsgemäßen Verbindungen zu einem nützlichen Wirkstoff für wässrige Formulierungen zum Insektenschutz, da die traditionell zum Insektenschutz eingesetzten Verbindungen prinzipiell wasserunlöslich oder nur in begrenztem Maße wasserlöslich sind.

Tabelle 1 zeigt die Wasserlöslichkeit der erfindungsgemäßen Verbindung PMD-S (*para-*Menthane-3,8-diol Succinat) im Vergleich zu üblichen in Insektenschutzmitteln eingesetzten Wirkstoffen.

**Tabelle 1**

| **Wirkstoff** | **Löslichkeit in Wasser (g/L)** |
|---|---|
| DEET | 1 g/L bei 25°C |
| KBR 3023 | 8.2 g/L bei 20°C |
| PMD | 0.29 g/L bei 25°C |
| IR 3535 | 70 g/L bei 20°C |
| PMD-S | >250 g/L bei 25°C |

Die erfindungsgemäßen Verbindungen weisen generell auch eine hohe Löslichkeit in Chloroform, Aceton, Diethylether, Ethanol, Methanol, Dimethylsulfoxid und Acetonitril, aber nur beschränkte Löslichkeit in Alkanen, wie z.B. Pentan und Hexan, auf.

Die erfindungsgemäßen Verbindungen können ferner die Wasserlöslichkeit von PMD erhöhen, wie in Figur 2 am Beispiel des Dreikomponentensystem PMD/PMD-S/Wasser gezeigt ist. L markiert dabei den Bereich einphasiger Mischungen und N den Bereich mehrphasiger Mischungen. Wie dem Phasendiagramm zu entnehmen ist, besteht das Zweikomponentensystem H₂O/PMD (durch die untere Achse dargestellt) abgesehen von eventuellen Extremwerten im Bereich von nahezu 100% H₂O oder nahezu 100% PMD immer aus mehrere Phasen, jedoch kann durch die Zugabe einer ausreichenden Menge an PMD-S die Ausbildung einer einphasigen Mischung erreicht werden.

### Stabilität

Es könnte vermutet werden, dass insbesondere die erfindungsgemäßen Verbindungen, in denen R eine Acylgruppe darstellt, besonders basenempfindlich sind. Jedoch lassen an der Beispielverbindung PMD-S durchgeführte Tests eine hohe Stabilität sowohl in basischen als auch in sauren Medien erkennen. So liegt die Halbwertszeit von PMD-S bei pH 11 bei etwa 16 Tagen. Die erfindungsgemäßen Verbindungen können folglich problemlos sowohl pur als auch in wässriger Lösung bei geeignetem pH-Wert aufbewahrt werden.

### Wirksamkeit als Insektenschutzmittel

Sowohl die erfindungsgemäßen Verbindungen als auch sie enthaltende Zusammensetzungen, wie z.B. Lösungen, Cremes und Lotionen, weisen Schutzaktivität gegen Insekten auf und können daher als Insektenschutzmittel eingesetzt werden.

So zeigt z.B. Figur 1, dass eine wässrige Lösung von PMD-S beim Menschen eine ähnlich lang anhaltende Schutzaktivität gegen Moskitos (*Aedes aegypti*) wie alkoholische PMD- und DEET-Lösungen gleicher Konzentration aufweist.

### Zytotoxizität und Hautreizung

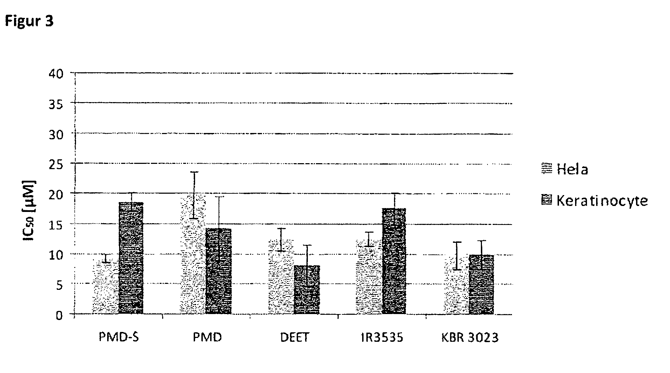

In Bezug auf den Einsatz der erfindungsgemäßen Verbindungen und sie enthaltende Zusammensetzungen als Insektenschutzmittel wurde ihre Zytotoxizität mit der Zytotoxizität gängiger Insektenschutzmittel verglichen. Hierzu wurden Zytotoxizitätsstudien an HeLa-Zellen und an Keratinozyten (SK-Mel-28) durchgeführt. Erstere Tests gelten als gute Alternative zu *in vivo* Draize Augenreizungstests, während letztere ein gutes Modell für die Hautkompatibilität sind. Als Beispiel für die erfindungsgemäßen Verbindungen dient hier wieder PMD-S. Die Ergebnisse sind in Tabelle 2 und Figur 3 zusammengefasst, wobei die IC₅₀-Werte die Konzentration des Wirkstoffes bei der 50 % der Zellen, bezogen auf unbehandelte Kontrollzellen, absterben bezeichnen.

**Tabelle 2**

| **insektenschutz-Wirkstoff** | **HeLa Zellen IC₅₀ [µMo]** | **Keratinocyten Zellen IC₅₀\|µMol]** |
|---|---|---|
| DEET | 12.39 ± 1.89 | 8.10 ± 3.41 |
| KBR3023 | 9.7 ± 2.25 | 9.82 ± 2,39 |
| PMI7 | 19.82 ± 3.89 | 14.18 ± 5.23 |
| IR *3535* | 12.49 ± 1.17 | 17.51 ± 2.76 |
| PMD-S | 928 ± 0.78 | 18.56 ± 1.56 |

Wie aus den Testergebnissen hervorgeht, ist PMD-S signifikant weniger toxisch als DEET oder KBR 3023 für Keratinozyten und vergleichbar in Toxizität mit PMD und IR 3535. Für HeLa Zellen ist PMD-S etwas toxischer als PMD, DEET und IR 3535 und ähnlich toxisch wie KBR 3023. Insgesamt ist also zu sehen, dass die Hautverträglichkeit der erfindungsgemäßen Verbindung besonders gut ist und die erwartete Augenreizung zwar etwas höher als die der gängigen Insektenschutzmittel ist, aber immer noch in derselben Größenordnung liegt.

Somit ist der Einsatz der erfindungsgemäßen Verbindungen in Insektenschutzmitteln oder anderen mit dem Körper in Kontakt kommenden Zusammensetzungen als unbedenklich in Bezug auf Hautreizungen und allergische Reaktionen anzusehen.

### Verwendung

Die erfindungsgemäßen *para*-Menthane-3,8-diolderivate können als Wirkstoffe in Insektenschutzmitteln eingesetzt werden. Dabei beträgt der Anteil der erfindungsgemäßen Verbindung typischerweise 1-40 Gew.-%, bevorzugter Weise 5 - 35 Gew.-% und am bevorzugtesten 10-30 Gew.-% bezogen auf das Gesamtgewicht.

Insbesondere wird es durch die Wasserlöslichkeit der erfindungsgemäßen Verbindungen zum ersten Mal möglich, konzentrierte Insektenschutzformulierungen auf Wasserbasis ganz ohne Zusatz von lösenden oder emulgierenden Additiven (wie z.B. Tenside oder Co-Lösungsmittel) herzustellen. In der Tat ist es sogar möglich, PMD mit in die Formulierung einzubeziehen, da die erfindungsgemäßen Verbindungen die Wasserlöslichkeit dieses Wirkstoffes erhöhen.

Ferner können die erfindungsgemäßen para-Menthane-3,8-diolderivate verwendet werden, um die Wasserlöslichkeit gängiger Insektenschutzmittel, wie z.B. PMD, DEET, Icaridin und IR3535 zu erhöhen, wodurch der Einsatz von organischen Lösungsmitteln und/oder Tensiden auch bei der Formulierung von Schutzmitteln auf Basis dieser Wirkstoffe reduziert oder gar vermieden werden kann. Eine entsprechende wässrige Lösung beinhaltet typischerweise die oben angegebene Menge der erfindungsgemäßen Verbindungen sowie 1-30 Gew.-%, bevorzugter Weise 5 - 25 Gew.-%, des weiteren Insektenschutzmittels bezogen auf das Gesamtgewicht.

### Herstellung

Die erfindungsgemäßen Verbindungen können durch Ether- oder Esterbildung aus PMD (para-Menthan-3,8-diol) und einer Alkyl- oder Acylverbindung hergestellt werden.

PMD kann durch die Zyklisierung von (+)-Citronellal, der Hauptkomponente von *Eucalyptus citriodora-*Öl*,* gewonnen werden und stellt somit einen kostengünstigen und nachhaltigen Rohstoff dar.

Bei der Alkyl- oder Acylverbindung kann es sich um eine Verbindung R-X handeln, wobei R eine gesättigte oder ungesättigte C₂-C₆ Alkyl- oder Acylgruppe ist und es sich bei X um ein Halogenatom, ―OH oder -OTs handelt. R ist mit mindestens einer funktionellen Gruppe ausgewählt aus ―CO₂H, ―SO₃ ―PO₄H und -NH₃⁺X⁻, welche geschützt, ungeschützt, als Anhydrid oder als Salz vorliegen kann, substituiert. Alternativ kann es sich bei der Acylverbindung auch um ein Carbonsäureanhydrid, wie z.B. Bernsteinsäureanhydrid, handeln. Ein solches Carbonsäureanhydrid kann ebenfalls mit einer funktionellen Gruppe ausgewählt aus ―CO₂H, ―SO₃H, ―PO₄H und ―NH₃⁺X, welche geschützt, ungeschützt oder als Salz vorliegen kann, substituiert sein.

Bei entsprechender Auswahl der erfindungsgemäßen Verbindung kann auch die Alkyl- oder Acylverbindung auf einem nachhaltigen und kostengünstigen Rohstoff basieren, so dass ausschließlich aus nachhaltigen Rohstoffen hergestellte Ausgangsmaterialien zum Einsatz kommen. So kann z.B. PMD-S durch Umsetzung von PMD mit Bernsteinsäureanhydrid, welches aus durch Fermentierung von vegetarischen Rohstoffen gewonnener Bernsteinsäure erhalten werden kann, hergestellt.

Eine solche Herstellung der erfindungsgemäßen Verbindungen durch leicht ausführbare chemischen Umsetzungen aus ausschließlich nachwachsenden Rohstoffen erlaubt auch eine Produktion vor Ort in Gebieten, in denen Krankheitsübertragungen durch Mücken oder andere Insekten ein besonderes Problem darstellen.

Die Herstellung der erfindungsgemäßen Verbindungen erweist sich als relativ kostengünstig im Vergleich zu bestehenden Produkten, da die Veretherung oder Veresterung von PMD großtechnisch und relativ kostengünstig durchgeführt werden kann und PMD, wie oben erwähnt, aus *Eucalyptus citriodora-*Öl*,* einem der billigsten ätherischen Öle überhaupt gewonnen werden. Auch Produktformulierungen auf Basis der erfindungsgemäßen Verbindungen weisen einen Kostenvorteil gegenüber bisher verwendeten Produkten auf, da weder Tenside noch oder andere Lösungsvermittler für die Formulierungen benötigt werden.

Produktformulierungen auf Basis der erfindungsgemäßen Verbindungen können Additive wie z.B. Konservierungsmittel, Gelbildner und Parfüme enthalten.

### Bestimmung der Wirkdauer

Die Wirkdauer wurde entsprechend der folgenden Standardmethode bestimmt:
U.S. Environmental Protection Agency. Product Performance Test Guidelines. OPPTS 810.3700. Insect Repellants for Human Skin and Outdoor Premise. Public Draft (2000).

### Beispiele

### Umsetzung von PMD mit Bernsteinsäureanhydrid:

In einem Rundkolben wurden 13,1g (0,13 mol) Bernsteinsäureanhydrid und 22,5g (0.13 mol) PMD (Takasago International Corporation; Diastereomerengemisch bezüglich der C₈ Position; (+)-cis-PMD zu (-)-trans-PMD Verhältnis con 62 zu 38) und 65 mL Toluol vorgelegt. Die Mischung wurde in einer Argonatmosphäre für 8 Std. gerührt und unter Rückfluss erhitzt, während der Reaktionsverlauf gaschromatographisch überwacht wurde. Anschließend wurde das Toluol unter vermindertem Druck entfernt und der Rückstand aus Methanol umkristallisiert. Ausbeute: 84% reines PMD-S.

### Umsetzung von PMD-S mit Natriummethoxid:

In einem Rundkolben wurden 27,24g (0,1 mol) PMD-S, gelöst in 140 mL Methanol, vorgelegt. Anschließend wurden 5,4g (0,1 mol) Natriummethoxid in 200 mL Methanol gelöst und die Lösung unter Rühren tropfenweise zu der durch ein Eisbad gekühlten PMD-S Lösung gegeben. Nach beendeter Zugabe wurde das Methanol unter vermindertem Druck entfernt und der Rückstand aus Pentan/Diethyläther (2/1) umkristallisiert. Ausbeute: 87% des Natriumsalzes von PMD-S.

### Formulierungsbeispiel 1:

Eine wässrige Zusammensetzung eines Insektenschutzmittels wurde durch Mischen der in der folgenden Tabelle aufgeführten Komponenten in dem angegebenen Verhältnis erhalten.

| **Gew:%** | **Komponente** |
|---|---|
| 78,85 | Wasser |
| 20,00 | PMD-S |
| 0,50 | Duftstoffe |
| 0,40 | Natriumbenzoat |
| 0,15 | Kaliumsorbat |
| 0,10 | Tocopherol |

### Formulierungsbeispiel 2:

Eine wässrige Zusammensetzung eines Insektenschutzmittels wurde durch Mischen der in der folgenden Tabelle aufgeführten Komponenten in dem angegebenen Verhältnis erhalten.

| **Gew.-%** | **Komponente** |
|---|---|
| 68,85 | Wasser |
| 25,00 | PMD-S |
| 5,00 | PMD |
| 0,50 | Duftstoffe |
| 0,40 | Natriumbenzoat |
| 0,15 | Kaliumsorbat |
| 0,10 | Tocopherol |

## Patentansprüche

1. Verbindung gemäß der folgenden Formel (1): 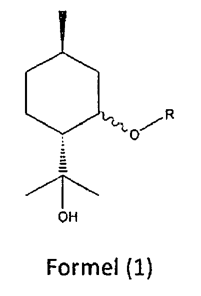 wobei R eine gesättigte oder ungesättigte C₂-C₆ Alkyl- oder Acylgruppe ist, welche mit mindestens einer funktionellen Gruppe ausgewählt aus -CO₂M, -SO₃M, -PO₄M und -NH₃⁺X⁻ substituiert ist, M Wasserstoff oder ein Alkalimetall darstellt und X⁻ ein Halogenid ist.

2. Verbindung gemäß Anspruch 1, wobei es sich um eine Verbindung der folgenden Formel (3) handelt: 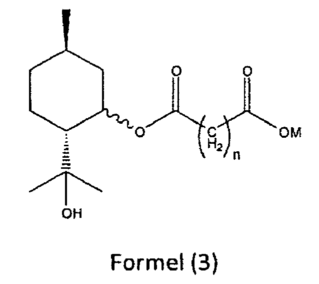 wobei n einen Wert von 1 bis 4 annehmen kann und M Wasserstoff oder ein Alkalimetall darstellt.

3. Verbindung gemäß Anspruch 2, wobei es sich um eine Verbindung der folgenden Formel (4) handelt: 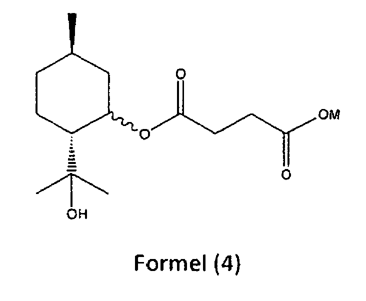 wobei M Wasserstoff oder Natrium darstellt.

4. Wässrige Lösung, umfassend eine oder mehrere Verbindungen gemäß einem der Ansprüche 1-3.

5. Wässrige Lösung gemäß Anspruch 4, wobei der Anteil der Verbindung gemäß einem der Ansprüche 1-3 1-40 Gew.-% bezogen auf das Gesamtgewicht beträgt.

6. Wässrige Lösung gemäß Anspruch 4 oder Anspruch 5, welche ferner ein oder mehrere Additive ausgewählt aus Konservierungsstoffen, Gelbildnern und Parfümen enthält.

7. Wässrige Lösung gemäß einem der Ansprüche 4 bis 6, welche ferner ein weiteres Insektenschutzmittel enthält.

8. Wässrige Lösung gemäß Anspruch 7, wobei das weitere Insektenschutzmittel aus der Gruppe para-Menthan-3,8-diol (PMD), N,N-Diethyl-m-methylbenzamid (DEET), 1-(1-Methylpropoxycarbonyl)-2-(2-hydroxyethyl)piperidin (Icaridin) und Ethyl-3-[(acetyl)(butyl)amino]propanoat (IR3535) ausgewählt ist.

9. Wässrige Lösung gemäß Anspruch 7 oder Anspruch 8, wobei der Anteil des weiteren Insektenschutzmittels 1-30 Gew.-% bezogen auf das Gesamtgewicht beträgt.

10. Verfahren zur Herstellung einer Verbindung gemäß einem der Ansprüche 1-3, umfassend die Umsetzung einer Verbindung der folgenden Formel (2) 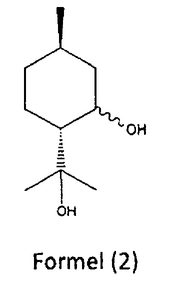 mit einer Verbindung R-A, wobei R wie in Anspruch 1 definiert ist und A ein Halogenatom, -OH oder-OTs darstellt (Ts =Toluolsulfonyl).

11. Verfahren zur Herstellung einer Verbindung gemäß einem der Ansprüche 2 und 3, umfassend die Umsetzung einer Verbindung der folgenden Formel (2) 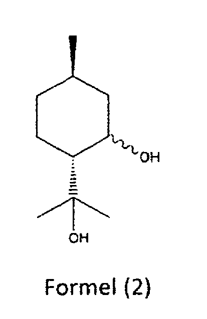 mit einer Carbonsäure oder einem Carbonsäurederivat.

12. Verfahren zur Herstellung einer Verbindung gemäß Anspruch 3, umfassend die Umsetzung einer Verbindung der Formel (2) 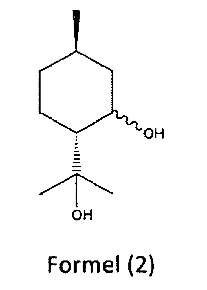 mit Bernsteinsäure oder einem Bernsteinsäurederivat.

13. Verfahren gemäß Anspruch 12, wobei die Verbindung der Formel (2) mit Bernsteinsäureanhydrid umgesetzt wird.

14. Verwendung einer Verbindung gemäß einem der Ansprüche 1 bis 3 zur Abwehr von Insekten.

15. Verwendung einer wässrigen Lösung gemäß einem der Ansprüche 4 bis 9 zur Abwehr von Insekten.

16. Verwendung einer Verbindung gemäß einem der Ansprüche 1 bis 3 zur Erhöhung der Wasserlöslichkeit von Insektenschutzmitteln.

17. Verwendung gemäß Anspruch 16, wobei das Insektenschutzmittel ausgewählt ist aus der Gruppe *para*-Menthan-3,8-diol (PMD), *N,N*-Diethyl-*m*-methylbenzamid (DEET), 1-(1-Methylpropoxycarbonyl)-2-(2-hydroxyethyl)piperidin (Icaridin) und Ethyl-3-[(acetyl)(butyl)amino]propanoat (IR3535).

## Claims

1. Compound according to the following formula (1): 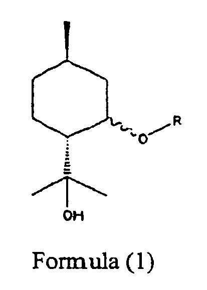 wherein R is a saturated or unsaturated C₂-C₆ alkyl group or acyl group, which is substituted by at least one functional group selected from -CO₂M, -SO₃M, -PO₄M and -NH₃+X⁻, M represents hydrogen or an alkali metal and X⁻ is a halide.

2. Compound according to claim 1, wherein it is a compound of the following formula (3)_{:} 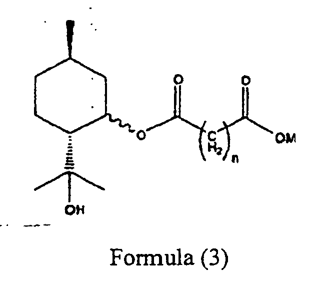 wherein n may assume a value from 1 to 4 and M represents hydrogen or an alkali metal.

3. Compound according to claim 2, wherein it is a compound of the following formula (4): 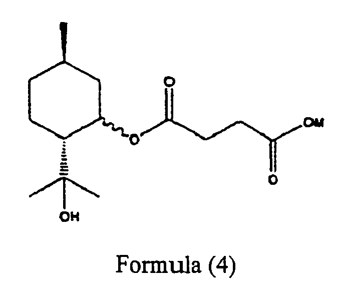 wherein M represents hydrogen or sodium.

4. Aqueous solution comprising one or more compounds according to one of claims 1-3.

5. Aqueous solution according to claim 4, wherein the proportion of compound according to one of claims 1-3 is 1-40 wt.% relative to the total weight.

6. Aqueous solution according to claim 4 or claim 5, which also contains one or more additives selected from preservatives, gelatinising agents and perfumes.

7. Aqueous solution according to one of claims 4 to 6, which also contains a further insect repellent.

8. Aqueous solution according to claim 7, wherein the further insect repellent is selected from the group para-menthane-3,8-diol (PMD), N,N-diethyl-m-methylbenzamide (DEET), 1-(1-methylpropoxycarbonyl)-2-(2-hydroxyethyl)piperidine (Icaridin) and ethyl-3-[(acetyl)(butyl)amino]propanoate (IR3535).

9. Aqueous solution according to claim 7 or claim 8, wherein the proportion of the further insect propellant is 1-30 wt.% relative to the total weight.

10. Method for producing a compound according to one of claims 1-3, comprising the reaction of a compound of the following formula (2) 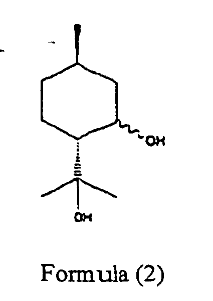 with a compound R-A, wherein R is defined as in claim 1 and A represents a halogen atom, -OH or -OTs (Ts = toluenesulphonyl).

11. Method for producing a compound according to one of claims 2 and 3, comprising the reaction of a compound of the following formula (2) 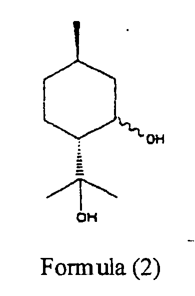 with a carboxylic acid or a carboxylic acid derivative.

12. Method for producing a compound according to claim 3, comprising the reaction of a compound of formula (2) 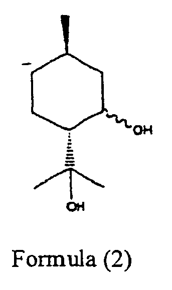 with succinic acid or a succinic acid derivative.

13. Method according to claim 12, wherein the compound of formula (2) is reacted-wit succinic anhydride.

14. Use of a compound according to one of claims 1 to 3 for protection against insects.

15. Use of an aqueous solution according to one of claims 4 to 9 for protection against insects.

16. Use of a compound according to one of claims 1 to 3 to increase the solubility in water of insect repellents.

17. Use according to claim 16, wherein the insect repellent is selected from the group para-menthane-3,8-diol (PMD), N,N diethyl-m-methylbenzamide (DEET), 1-(1-methylpropoxycarbonyl)-2-(2-hydroxyethyl)piperidine (Icaridin) and ethyl-3-[(acetyl)(butyl)amino]propanoate (IR3535).

## Revendications

1. Composé selon la formule (1) suivante : 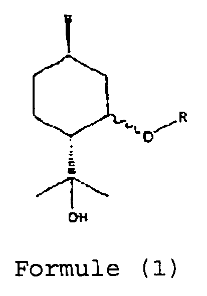 dans laquelle R est un groupe acyle ou alkyle en C₂-C₆ saturé ou insaturé, qui est substitué par au moins un groupe fonctionnel constitué par -CO₂M, -SO₃M, -PO₄M et -NH₃⁺X⁻, M représente un atome d'hydrogène ou un métal alcalin et X⁻ est un atome d'halogénure.

2. Composé selon la revendication 1, dans lequel il s'agit d'un composé de formule (3) suivante : 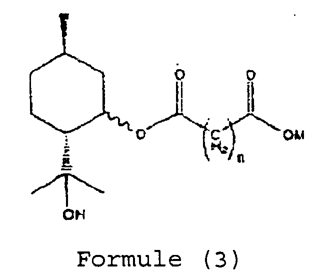 dans laquelle n peut prendre une valeur de 1 à 4 et M représente un atome d'hydrogène ou un métal alcalin.

3. Composé selon la revendication 2, dans lequel il s'agit d'un composé de formule (4) suivante : 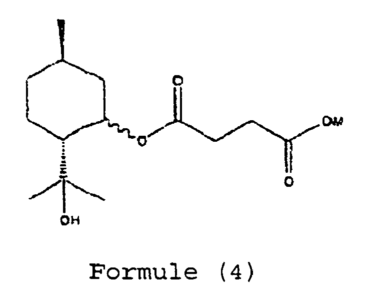 dans laquelle M représente un atome d'hydrogène ou de sodium.

4. Solution aqueuse comprenant un ou plusieurs composés selon l'une des revendications 1 à 3.

5. Solution aqueuse selon la revendication 4, dans laquelle la proportion du composé selon l'une des revendications 1 à 3 atteint 1 à 40 % en poids, par rapport au poids total.

6. Solution aqueuse selon la revendication 4 ou 5, qui comprend en outre un ou plusieurs additifs choisis parmi les conservateurs, les gélifiants et les parfums.

7. Solution aqueuse selon l'une des revendications 4 à 6, qui comprend en outre un autre répulsif contre les insectes.

8. Solution aqueuse selon la revendication 7, dans laquelle l'autre répulsif contre les insectes est choisi dans le groupe constitué par le para-menthane-3,8-diol (PMD), le *N,N*-diéthyl-*m*-méthylbenzamide (DEET), la 1-(1-méthylpropoxy-carbonyl)-2-(2-hydroxyéthyl)pipéridine (icaridine) et le 3-[(acétyl)(butyl)amino]propanoate d'éthyle (IR3535).

9. Solution aqueuse selon la revendication 7 ou la revendication 8, dans laquelle la proportion de l'autre répulsif contre les insectes atteint de 1 à 30 % en poids par rapport au poids total.

10. Procédé de préparation d'un composé selon l'une des revendications 1 à 3, comprenant la conversion d'un composé de formule (2) suivante : 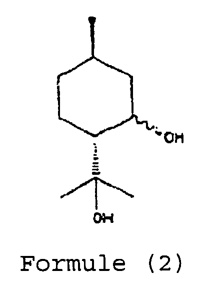 avec un composé R-A, dans lequel R est tel que défini dans la revendication 1 et A représente un atome d'halogène, -OH ou -OTs (Ts = toluènesulfonyle).

11. Procédé de préparation d'un composé selon l'une des revendications 2 et 3, comprenant la conversion d'un composé de formule suivante (2) 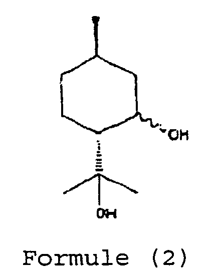 avec un acide carboxylique ou un dérivé d'acide carboxylique.

12. Procédé de préparation d'un composé selon la revendication 3, comprenant la conversion d'un composé de formule (2) 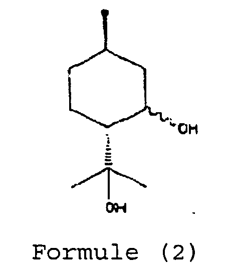 avec un acide succinique ou un dérivé d'acide succinique.

13. Procédé selon la revendication 12, dans lequel le composé de formule (2) est converti avec un anhydride d'acide succinique.

14. Utilisation d'un composé selon l'une des revendications 1 à 3 pour la protection contre les insectes.

15. Utilisation d'une solution aqueuse selon l'une des revendications 4 à 9 pour la protection contre les insectes.

16. Utilisation d'un composé selon l'une des revendications 1 à 3 pour augmenter la solubilité dans l'eau des répulsifs contre les insectes.

17. Utilisation selon la revendication 16, dans laquelle le répulsif contre les insectes est choisi dans le groupe constitué par le para-menthane-3,8-diol (PMD), le N,N-diéthyl-m-méthylbenzamide (DEET), la 1-(1-méthylpropoxycarbonyl)-2-(2-hydroxyéthyl)pipéridine (icaridine) et le 3-[(acétyl)(butyl)amino]propanoate d'éthyle (IR3535).
